(19) Europäisches Patentamt / European Patent Office / Office européen des brevets

(11) **EP 4 493 915 B1**

(12) **EUROPEAN PATENT SPECIFICATION**

(45) Date of publication and mention of the grant of the patent:
**27.05.2026   Bulletin 2026/22**

(21) Application number: **23769391.6**

(22) Date of filing: **14.03.2023**

(51) International Patent Classification (IPC):
**G01N 21/27** (2006.01)    **G01J 3/28** (2006.01)
**G01J 3/10** (2006.01)

(52) Cooperative Patent Classification (CPC):
**G01N 21/31; G01J 3/10; G01J 3/2823;**
**G01N 21/474; G01N 21/4795; G01N 33/085;**
G01N 2021/4742; G01N 2201/0826

(86) International application number:
**PCT/CA2023/050329**

(87) International publication number:
**WO 2023/173208 (21.09.2023 Gazette 2023/38)**

(54) **SYSTEM OF EGG FERTILITY AND GENDER DETECTION**

SYSTEM ZUR EIFERTILITÄTS- UND GESCHLECHTSDETEKTION

SYSTÈME DE DÉTECTION DE FERTILITÉ D'OEUFS ET DE SEXE

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**

(30) Priority:  **14.03.2022   US 202263319539 P**

(43) Date of publication of application:
**22.01.2025   Bulletin 2025/04**

(73) Proprietor: **Matrixspec Solutions Inc.**
**Baie D'Urfé, QC H9X 3V1 (CA)**

(72) Inventors:
• **LIU, Li**
**Baie D'Urfe, Québec H9X 3V1 (CA)**
• **NGADI, Michael**
**Baie D'Urfe, Québec H9X 3V1 (CA)**

(74) Representative: **Barker Brettell LLP**
**100 Hagley Road**
**Edgbaston**
**Birmingham B16 8QQ (GB)**

(56) References cited:
EP-A1- 4 260 048          CA-A1- 2 804 903
CN-U- 214 041 128          KR-A- 20180 055 970
US-A- 5 615 777          US-A1- 2020 110 068

## Description

## FIELD

The present disclosure generally relates to a system of detecting egg fertility and gender.

## INTRODUCTION

Fertility and hatchability of eggs are critical economic factors for hatcheries and poultry breeding farms. Only about 60 to 90% of incubated eggs are fertile and eventually hatch in commercial hatcheries. Non-hatching eggs include infertile eggs or fertile eggs where the embryos have died. Infertile eggs may comprise up to 25% of all eggs set. These eggs may be useful for commercial egg tables or low grade food stock.

The sex of fertile eggs is also among the egg characteristics of interest for the poultry industry. In the layer egg industry, chicks are sexed at hatch and the female birds (that will lay eggs) are considered paramount while the male birds are culled. The opposite is the case with the broiler industry in which the male species are crucial. In either case, discarding of the unwanted chicks creates serious bottlenecks as far as waste disposal and animal welfare issues are concerned. CN 214 041 128 discloses an egg quality on-line detection device based on transmission-type hyperspectral imaging. The egg quality on-line detection device comprises a transmission-type hyperspectral imaging module, an on-line transmission module and an upper computer, the upper computer is used for controlling the transmission-type hyperspectral imaging module to achieve hyperspectral imaging, collecting images and controlling the online transmission module. A hyperspectral image of an egg can be detected by isolating an egg shell, so that the spectral characteristics of each part inside the egg can be further obtained, and the on-line rapid and nondestructive detection of the quality of the egg can be realized. KR 2018 0055970 discloses a method for determining the survival of an embryo in a fertile chicken egg using a hyperspectral image. The method comprises the steps of: (a) capturing an ultra-spectral transmission image of 400 to 1,000 nm from a fertile chicken egg irradiated by halogen illumination with an electron multiplying charge-coupled device (EMCCD) camera; and (b) performing a preprocessing method for obtaining a clear image, and determining the survival through a texture analysis after obtaining an image representing the inside of the fertile chicken egg through principal component analysis (PCA) from the image captured by the camera. CA 2 804 903 discloses hyperspectral method for detecting the present condition of an avian egg in which a neural network algorithm is used to compare the spectrum of a test egg against a spectral library. EP 4 260 048 discloses an egg analysis system and a computer implemented method of analysing eggs. The system comprises a light source for illuminating an egg, a photon detector for detecting a fluctuation of scattering light emitted from the egg, a processor, and a memory storing instructions which when executed by the processor configure the processor to receive scattering light data from the photon detector, digitize the scattering light data, and analyze the digitized scattering light data. The method comprises receiving angle, frequency and intensity data of scattering light from an egg illuminated using a light source, identifying a germinal disc in the egg from the scattering light data, and determining at least one of a fertility or a sex of the egg based on the size and structure of the germinal disc.

## SUMMARY

In accordance with an aspect, there is provided an egg analysis system as claimed in claim 1. The system comprises system comprises at least one light source for illuminating an egg, by emitting light that covers the field of view of a hyperspectral imaging camera, the hyperspectral imaging camera for detecting a fluctuation of scattering light emitted from the egg, at least one processor, and a memory storing instructions which, when executed by the at least one processor, configure the at least one processor to actuate the at least one light source to illuminate the egg, receive, from the hyperspectral imaging camera, scattering light data comprising angle, frequency and intensity data, digitize the scattering light data, analyse the digitised scattering light data to identify features of material present, compare the identified features of material present with known features for germinal discs, to identify the germinal disc in the light scattering data; and determine at least one of a fertility or a sex of the egg based on the size and structure of the germinal disc.

The light source may emit light at one of approximately 860 nm, 1050 nm or 1250 nm.

In this respect, before explaining at least one embodiment in detail, it is to be understood that the embodiments are not limited in application to the details of construction and to the arrangements of the components set forth in the following description or illustrated in the drawings. Also, it is to be understood that the phraseology and terminology employed herein are for the purpose of description and should not be regarded as limiting.

## DESCRIPTION OF THE FIGURES

Embodiments will be described, by way of example only, with reference to the attached figures, wherein in the figures:

FIG. 1A illustrates, in a component diagram, an example of a system for analyzing eggs, in accordance with some embodiments;

**FIG. 1B** illustrates, in a component diagram, another example of a system for analyzing eggs in accordance with some embodiments;

**FIG. 2A** illustrates, in a schematic drawing, an example of a system with optical fiber with incident beam angle for acquiring scattering images of eggs, in accordance with some embodiments;

**FIG. 2B** illustrates, in a schematic drawing, an example of a system with LED line light for acquiring scattering images of eggs, in accordance with some embodiments;

**FIGs. 3A to 3E** illustrate scan images of a germinal disc in an egg, in accordance with some embodiments;

**FIG. 4** illustrates, in a flowchart, an example of a method of analyzing eggs, in accordance with some embodiments;

**FIG. 5** illustrates, in a flowchart, an example of a protocol (e.g., method) of handling an egg such that its germinal disc is located on the top (large end) of the egg, in accordance with some embodiments;

**FIG. 6A** illustrates an example of eggs positioned at 45 degree angle, in accordance with some embodiments;

**FIG. 6B** illustrates an example of eggs positioned in a vertical position, in accordance with some embodiments;

**FIG. 7** illustrates, in a flowchart, an example of a method of analyzing light scattering, in accordance with some embodiments; and

**FIG. 8** is a schematic diagram of a computing device such as a server or other computer in a device.

It is understood that throughout the description and figures, like features are identified by like reference numerals.

## DETAILED DESCRIPTION

Embodiments of methods, systems, and apparatus are described through reference to the drawings. Applicant notes that the described embodiments and examples are illustrative and non-limiting. Practical implementation of the features may incorporate a combination of some or all of the aspects, and features described herein should not be taken as indications of future or existing product plans.

In some embodiments, in order to identify and isolate infertile eggs and separate female and male eggs, in-ovo fertility and gender determination processes have been developed using Hyperspectral Imaging (i.e., time-resolved hyperspectral imaging, spatially-resolved hyperspectral imaging), Super-Resolution hyperspectral image processing and analysis, Machine Learning and Deep Learning technologies. The processes were developed based on statistical methods and have shown very promising results on egg fertility and gender determination prior to incubation. Such in-ovo fertility and gender detection systems may include the acquisition of good quality hyperspectral images where the light source may play a role.

In some embodiments, in-ovo fertility and gender determination techniques have been developed based on light scattering patterns through the pre-incubated eggs. The scattered light patterns were then analyzed using gray-level co-occurrence matrix, statistical methods, and Fourier analysis in order to determine the egg fertility and gender of the eggs.

In some embodiments, 'distinguishing features' in the obtained hyperspectral images and/or light scattering patterns have been identified that can consistently differentiate non-fertile eggs from fertile eggs, and male eggs from female eggs.

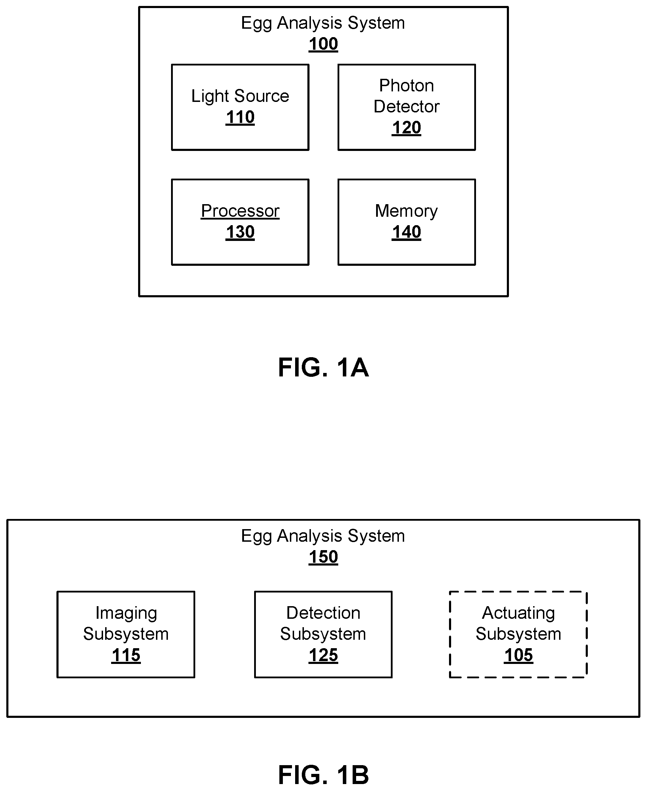

**FIG. 1A** illustrates, in a component diagram, an example of a system for analyzing eggs 100, in accordance with some embodiments. The system **100** comprises a light source (e.g., a laser) **110,** and at least one photon detector **120**. In some embodiments, eggs may be illuminated by a laser **110** beam (or other light source beam), and the fluctuations of the scattered light detected at a known scattering angle θ by a fast photon detector **120** (e.g., streak camera). Cells in the eggs scatter the light from the laser **110** beam, and the imprint information is detected by the photon detector **120** and used in an analysis that yields information about the cellular particles. The intensity fluctuations of the incident beam is characterized by computing the intensity correlation function, whose analysis provides the diffusion coefficient of the particles (also known as diffusion constant). Since particles of different sizes scatter with different intensities, different scattering angles may be examined in order to determine the optimum angle of detection for the eggs. Other components may be added to the system **100,** including a processor **130** and a memory **140** storing instructions to digitize and analyze the imprint information. For example, the memory **140** may include a digitization module and an analysis module.

**FIG. 1B** illustrates, in a component diagram, another example of a system for analyzing eggs **150,** in accordance with some embodiments. The system **150** includes an imaging subsystem **115,** a detection subsystem **125,** and optionally an actuating subsystem **105**. In some embodiments, the actuating subsystem **105** may be external to the system **150**. The subsystems **115, 125** and **105** may have separate processors **130** and memory **140,** or alternatively may share a common system **150** processor **130** and memory **140**.

In some embodiments, a light source for an in-ovo fertility and gender detection system **100, 150** may provide uniform illumination with high intensity to the eggs within the field of view (FOV) of the hyperspectral imaging camera. It should be noted that intensity (also called 'Radiance') is radiant flux emitted, reflected, transmitted or received by a surface, per unit solid angle per unit projected area, while spectral intensity (also called 'spectral radiance') is radiance of a surface per unit wavelength. Intensity is the integral of spectral intensity over the wavelengths of the light source. The following lists more specific specifications for a light source:

1. A light that can cover the field of view (FOV) of the hyperspectral camera (e.g., approximately 16 centimeters (cm) x 2.5 cm, or other dimensions as desired)

2. High spectral intensity at the interested wavelengths range
(e.g., at least 120 mW·sr$^{-1}$·cm$^{-2}$·nm$^{-1}$, or other spectral radiance as desired)

3. Uniform illumination for each egg within the FOV (e.g., variance < 10%, or as desired)

4. A 'cool' light that produce little heat for a long time run

In addition, the light source may also meet the following specifications in order to be appropriate for industrial use:

5. High stability for a long time run (e.g., 8 hrs or above, or as desired)

6. Long lifetime of the lamp (e.g., >1,000 hrs, or as desired)

In some embodiments, the light source may be part of an imaging subsystem **115, 200, 250** of a detection system **100, 150** that includes a line scan spectrograph interconnected with an InGaAs camera (i.e., a hyperspectral imaging camera in the short-wave infrared range), a digital controlled conveyor, and a custom-designed egg holder. The imaging subsystem **115, 200, 250** works in a transmission mode. Eggs are placed in the egg holder and on the conveyor. The light source in the imaging subsystem is positioned to align with the hyperspectral imaging camera and to provide back illumination for an egg to facilitate the acquisition of hypercube. The light source can be developed based on different lights as long as they are able to meet the above-said specifications.

In some embodiments, the light source is part of an imaging subsystem **115, 200** that includes a line-scan spectrograph interconnected with an InGaAs camera (i.e., configured to capture images (e.g., spectral images, scattered light). In one example implementation, the spectrograph with a near-infrared spectral range spanning approximately 900 nm to 1700 nm and a spectral resolution of 2.8 nm. In an embodiment, image data is collected in transmission mode. In an embodiment, image data is collected and processed at 100 frames per second. In an embodiment, the imaging system may include a wide field, area scan, snapshot camera.

In some embodiments, one or more light sources **110** in the imaging subsystem **115** may be used to provide back illumination for an egg to facilitate image capture of the fluctuations of scattered light. In one example implementation, a single 250-watt quartz tungsten halogen lamp is used as a light source. Such light source may be connected to an optical fiber (e.g., liquid light guide) to control incident beam size. The controllable beam size and incident beam position using an optical fiber will enhance the precision of algorithm to determine the presence and structure of germinal disc.

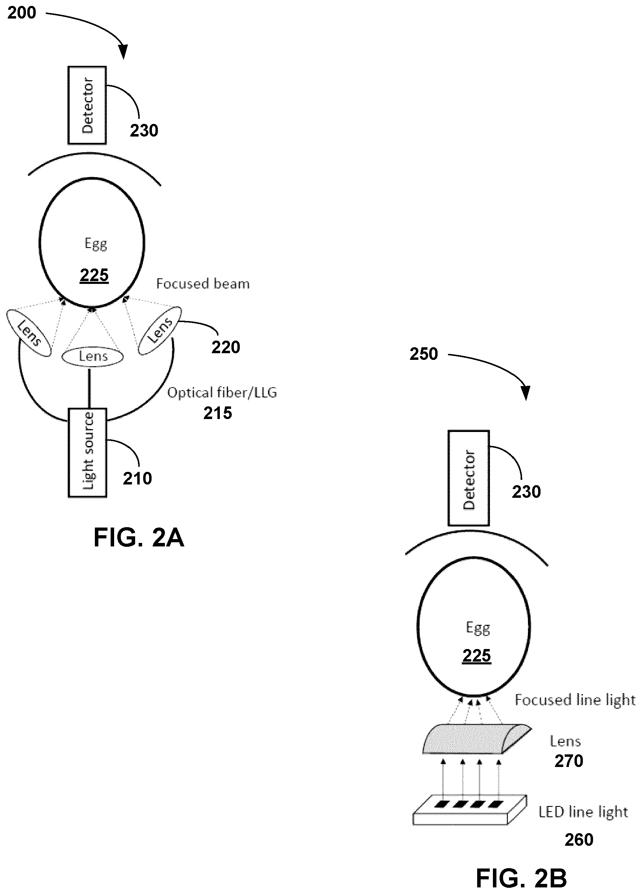

**FIG. 2A** illustrates, in a schematic drawing, an example of a system with optical fiber with incident beam angle for acquiring scattering images of eggs **200,** in accordance with some embodiments. The system comprises a light source **210** connected to one or more lenses **220** via optical fiber/LLG **215,** and a detector **230** receiving the focused beam that has been passed through an egg **225.**

In some embodiments, the light sources **110** may consist of an optical fiber (or a liquid light guide, LLG) and/or a focusing lens to control the size of light beam and incident beam position to generate good scattering images. The size of light beam is critical for the illumination system. A larger beam may give higher light throughput and a larger scattering area, but it can be difficult to quantify the light scattering characteristics. A smaller beam, albeit desirable for scattering measurement, but it may have lower efficiency for the camera that can result in lower SNR (i.e., signal-to-noise ratio) images. The selected beam size should be a trade-off between the light efficiency and scattering measurement. In one example implementation, a liquid light guide (LLG) with a core diameter of 7.6 mm and 0.52 numerical aperture is used to guide the incident light to the illumination position that may be right underneath the egg. A focusing lens may be placed between the end of LLG and the egg in order to form a sharp and focused incident beam at the egg. Multiple optical fibers and focusing lens may be used to provide higher light throughput in order to have a good balance between the light efficiency and scattering measurement. In this case, the incident angles of the fibers/LLGs may be adjusted within a small range (less than 30°) with equal intervals.

In some embodiments, an optimal light source based on halogen lamps (e.g., quartz tungsten halogen, QTH) may consist of 2 or 3 identical light source units each of which provide back illumination for a column of eggs. A QTH light source unit may include a 250 W QTH lamp, a light source housing, a lamp power supply, a fiber bundle focusing assembly, a liquid light guide, a light guide collimating probe, and all the necessary interconnection cables and parts for operation.

The QTH lamp may be operated in current, power, or intensity control mode to meet the operational requirements. The lamp power supply is highly regulated for maximum stability. The QTH housing offers a built-in ignitor board and rear reflector for optimum lamp performance. A built-in cooling fan maintains a safe operating temperature for the lamp. The fiber bundle focusing assembly that is connected to the light housing is used to convert a collimated light source into a fiber optic source, i.e., a liquid light guide (LLG) that is connected to the focusing assembly. The liquid light guide carries light from the housing to a position underneath the egg holder where it is aligned with the hyperspectral imaging camera. The light guide collimating probe that is connected to the LLG collimates the highly divergent outputs of LLG and provides back illumination for an egg to acquire high quality of hypercube.

**FIG. 2B** illustrates, in a schematic drawing, an example of a system with LED line light for acquiring scattering images of eggs **250,** in accordance with some embodiments. The system comprises an LED line light source **260,** a lense **270,** and the detector **230** receiving the focused beam that has been passed through the egg **225.**

Light-emitting diodes (LEDs) are semiconductors that convert electrical energy into light energy. Compared to QTH lamps, LED lamps can provide much higher light output intensity, need lower energy consumption, have much longer shelf life without decreasing light intensity, and produce much less heat. However, unlike a QTH lamp that produces a continuous spectrum of light from near ultraviolet to the infrared, there are only a few wavelengths that are available for LED lamps especially in the IR/SWIR range.

Several wavelengths that were identified to be useful for the egg fertility and gender determination include approximately 860 nm, 1050 nm, and 1250 nm. An LED line light was designed to accommodate the LED units with identified wavelengths at a carefully tuned ratio that can produce equal light output intensity level at all wavelengths. The spectral intensity of the light output, i.e., the radiance of a surface per unit wavelength should be at least:

$$120 \text{ mW} \cdot \text{sr}^{-1} \cdot \text{cm}^{-2} \cdot \text{nm}^{-1}.$$

Depending on the light output intensity level of LED units, the LED light source may work in strobe mode to overdrive LEDs to obtain a boost in their performance. If the LED light source works in strobe mode, the LEDs at all wavelengths should do strobing simultaneously. The LED line light should be mounted underneath the egg holder and aligned with the FOV of the HSI camera to provide back illumination for eggs to acquire good quality of hypercubes.

In some embodiments, the photon detector **120, 230** is part of a detection subsystem **125** that analyses received scattering data to detect cellular particles in an egg, including, for example, a germinal disc.

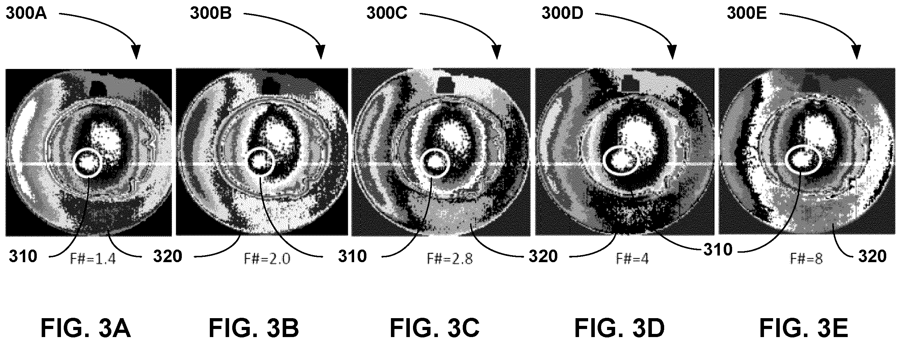

**FIGs. 3A to 3E** illustrate scan images **300A** to **300E** of a germinal disc **310** in an egg **320,** in accordance with some embodiments. Detection of the germinal disc **310** was performed in the light scattering images **300A** to **300E** that were obtained with different aperture sizes (F#) of the camera. The pattern of germinal disc **310** appeared as a round disc in light scattering images **300A** to **300E.** The egg handling protocol ensures that the germinal disc **310** moves to the top of the egg **320** in the field of view of the camera during scanning. Although the germinal disc **310** was able to be identified in all light scattering images, image **300C** with F# of 2.8 outperformed other scattering images with the best contrast and separability.

In some embodiments, system **100** may optionally include an actuating system **105** to actuate a conveyor configured to move an egg into the field of view of the system's photon detector **120, 230** (e.g., camera) optics. In one example implementation, the conveyor is a Dorner 2200 series conveyer system comprises a belt, a roller, a stepping motor, and a programmable logic controller (PLC) touch screen. The speed of the conveyor may be adjustable. For example, the speed of the conveyor may be adjusted based on the speed of the photon detector **120, 230** (e.g., the frame rate of camera optics) to minimize image distortion (e.g., motion blur). The speed of the conveyor may also be adjusted based on other factors, e.g., desired detection throughput, the working distance between detector and object, the desired illumination level at the object.

The conveyor may include trays or racks adapted to receive eggs therein. In some embodiments, the trays or rack may then be stored (either on or off the conveyor) while maintaining each egg in a given position (e.g., a vertical position, a first angled position, a second angled position, etc.). In some embodiments, the rack may rotate such that the eggs therein are maintained in a different positon (e.g., a vertical position, a first angled position, a second angled position, etc.).

In an embodiment, the conveyor may be configured to present multiple eggs (e.g., two eggs, four eggs, etc.) to be imaged simultaneously. Accordingly, in this embodiment, each spectral image or scattering data may include data for multiple eggs, and each such image or scattering data may be segmented during processing to isolate scattering data for each egg. Processor **130** may be configured to send control commands to conveyor to control its movement.

The imaging subsystem **115, 200, 250** may be interconnected with a detection subsystem **125** by way of a conventional serial or parallel interface. In an embodiment, imaging subsystem **115, 200, 250** may be interconnected with detection subsystem **125** by way of a network comprising wired links, wireless links, or a combination thereof. In this embodiment, one or both of imaging subsystem **115, 200, 250** and detection system **125** may include a suitable network interface and/or network transceivers. In some embodiments, the detection subsystem **125** may be a cloud service which receives scattering data for an egg as input, and provides a sex and/or fertility of the egg as output.

In some embodiments, the detection subsystem **125** or system **100** may connect to an actuating subsystem **105** to trigger actuation of apparatuses based on results computed by the detection subsystem **125.** The actuating subsystem **105** is operable to transmit a control signal to actuate an apparatus according to the classified unhatched egg. The actuating subsystem **105** is operable to generate data signals for the gender and fertility of the unhatched egg, for example. The actuating subsystem **100** is operable to transmit the output data signals to hardware or apparatus to trigger actuation thereof. For example, the actuating subsystem **105** may move or separate the unhatched egg.

In some embodiments, an actuating subsystem **105** may receive data signals of classification results from the detection subsystem **125** and removes the undesired eggs (non-fertile and/or male) from the assembly line using one or more apparatuses that are in physical contact with the eggs or otherwise can trigger movement or separation of eggs. For example, actuating subsystem **105** may include or interface with one or more robotic arms with end effectors (robotic hands) that may be used to grasp and drop or replace eggs which are indicated by the classification signals from detection subsystem as non-fertile and/or male eggs. There may be other apparatuses that can separate or move eggs based on the classification signals from detection subsystem and this is an illustrative example only. Accordingly, the actuating subsystem **105** triggers actuation of hardware components based on the classification signals from detection subsystem **125.** In example embodiments the actuation may involve physical movement of the eggs to separate the eggs into different streams, for example. As another example a conveyer may be triggered or controlled to move eggs. Detection subsystem **125** generates output signals for actuating subsystem **105** to provide control commands to trigger actuation of various apparatuses.

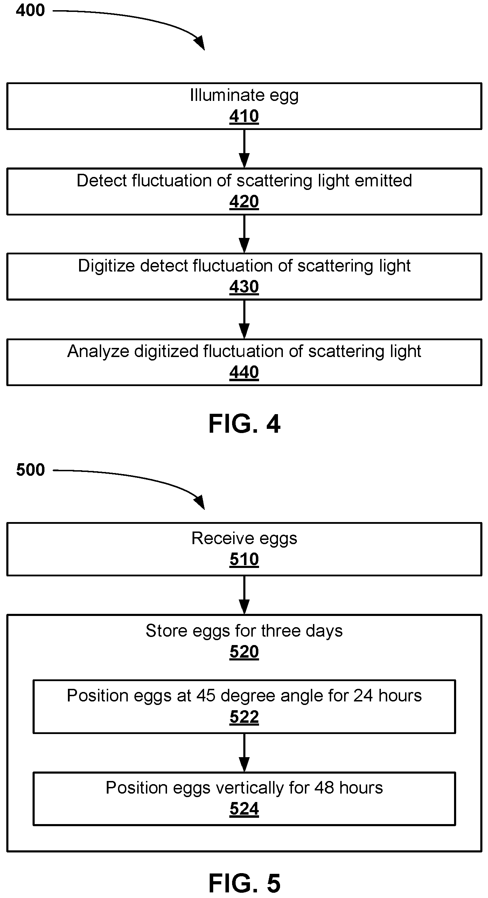

**FIG. 4** illustrates, in a flowchart, an example of a method of analyzing eggs **400,** in accordance with some embodiments. The method **400** comprises illuminating **410** an egg using a light source (e.g., a laser) **110** beam, and detecting **420** fluctuations of scattered light emitting from the egg using a fast photon detector **120.** Next the fluctuations, or imprint information, detected by the photon detector **120** is digitized **430,** and the digitized information analysed **440** for cellular particulars. For example, an intensity correlation function may be performed to determine the diffusion coefficients of the particles (i.e., diffusion constant). Other steps may be performed by the method **400,** including determining an optimal angle of detection for the egg.

The shape and size of the germinal disc which floats on the egg yolk is different between fertile and non-fertile eggs. The germinal disc in a fertile egg (also called blastoderm) is visually seen as a symmetrical circular ring, while the germinal disc in a non-fertile egg (also called blastodisc) looks like an asymmetrical solid spot. There have been several studies to identify possible gender differentiating features prior to incubation. It is known that there could be about 40,000 to 60,000 blastoderm cells available in the germinal disc of chicken egg. Female birds are heterogametic with one Z and one W sex chromosome, whereas male birds have two Z chromosomes. The Z chromosome has approximately threefold higher DNA content than the W chromosome. Therefore, the total DNA amount in cells from male birds will be higher than in cells from female birds.

Application of multivariate methods for spectral feature selection has produced promising results in such fields as tumor identification or classification of different cell types. Further, considering that the germinal disc of the male egg is denser than that of the female egg, the spectral scattering patterns are expected to be different and could explain the statistical difference observed by the spectral and image features extracted from the hyperspectral images. Therefore, the distinguishing features for fertility and gender detection may be discovered based on light scattering measurement.

Light scattering happens when light "hits" a small object (a particle or a molecule) and thereby changes its direction. The ability of scattering particles to scatter light depends on their densities and sizes. It is a process when incident light of energy is absorbed by an object and subsequently light of energy is emitted. The angle, frequency and intensity (i.e., power) of light scattering can be measured to determine the particle size and density of materials. Angle-resolved scattering measurements capture light as a function of the scattering angle, and invert the angles to deduce the average size of the scattering objects via a computational light scattering model such as Mie scattering theory, which predicts angles based on the size of the scattering sphere. Combining these techniques allows for a measurement of average scatter size and average particle size of the object. In addition, multiple scattering properties that can be calculated by averages of single sphere scattering efficiencies obtained from Mie scattering theory are also used to predict the structure of the object. Therefore, egg fertility and gender detection methods have been developed based on the predicted size and structure of germinal disc using different light scattering measurements and patterns.

The light scattering may vary with particle sizes and/or densities. The intensity of the scattered light in different directions may strongly depend on the wavelength of incident light and the size, shape and composition of particles. The effectiveness of scattering depends on the size parameter $x$ that is defined as $x = \frac{2\pi r}{\lambda}$, where r is the radius of a spherical particle (or particle size) and λ is the wavelength of incident light. A germinal disc normally having 40,000-60,000 blastoderm cells appears as either a single small white mass for unfertile eggs or a double ring and larger white disk for fertile eggs. The diameter of a germinal disc may vary from less than 1mm up to 4 mm. This indicates that the size parameter of a germinal disc is greater than 1,500, which means that the Mie scattering (for x=0.2 to 2000) or geometric optics (for x>2000) may be valid depending on the size of the GD. The light intensity in scattering image can be a function of the scattering angles and the angles can be inverted to deduce the average size of the scattering objects, i.e., the scattering size. The scattering size may also be measured with the scattering cross-section that can be determined by dividing the power of the scattered light by the intensity of the incident light.

As noted above, in some embodiments, prediction of egg fertility and gender of non-incubated eggs is based on identifying and characterizing light scattering patterns of the egg's germinal disc (or other cells in mitochondrial DNA). Therefore, the germinal disc should be in the camera's field of view (FOV) during scanning. Eggs may be scanned with its "big" (i.e., large) end upwards. The "big" or "large" end is considered to be the larger in diameter of the two ends of an egg. What is desired is to ensure that the germinal disc is located on top of the big/large end of the egg in order to appropriately record and measure its light scattering patterns. Normally the germinal disc in the egg could be located randomly anywhere on the surface of the egg's yolk. In some embodiments, a protocol of egg handling provides that the germinal disc is located on top of the egg (big/large end) and in the camera's field of view.

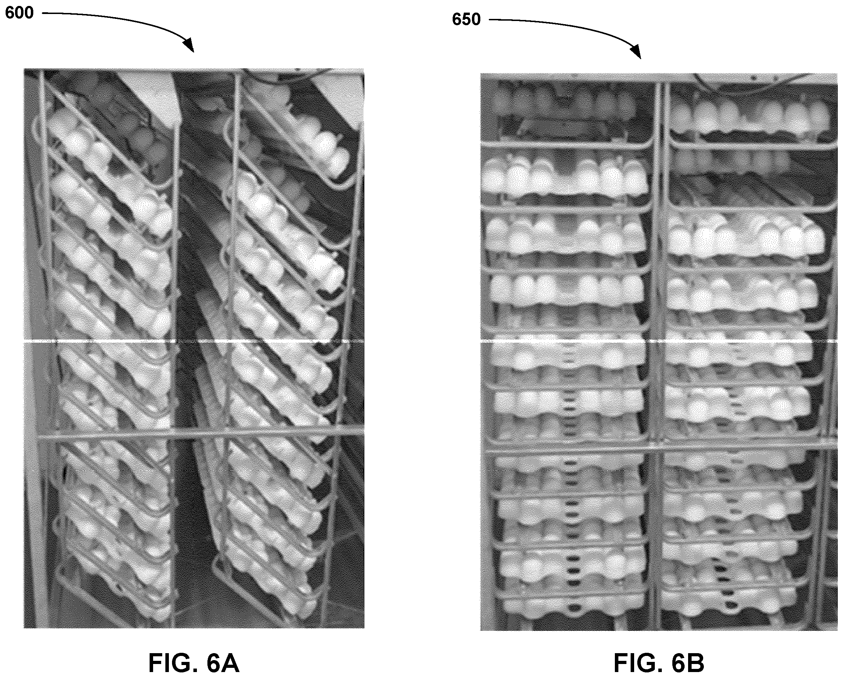

FIG. 5 illustrates, in a flowchart, an example of a protocol (e.g., method) of handling an egg such that its germinal disc is located on the top (large end) of the egg 500, in accordance with some embodiments. The method 500 may be performed by an egg processing system. Eggs may be placed in a tray 510 with their big end upwards. For example, an egg processing system may be configured to receive eggs in a manner that causes the eggs to fall into place in a rack of an egg holding apparatus. Next, the eggs may be stored 520 at approximately between 18 to 20 degrees Celsius for at least three days. The eggs (e.g., the rack holding the eggs) may be positioned 522 at a first angle (e.g., approximately a 45 degree angle) for a first period (e.g., the first approximately 24 hours). For example, an actuator of an egg processing system may rotate the rack at approximately a 45 degree angle. FIG. 6A illustrates an example of eggs positioned at 45 degree angle 600, in accordance with some embodiments. Next the eggs (e.g., the rack holding the eggs) are positioned 524 at a second angle (e.g., flat; the rack may be in a horizontal positon such that the eggs are in a vertical position with the "big" end up") for a second period (e.g., the next approximately 48 hours). For example, a timer may indicate that the rack has been at approximately 45 degrees for a first time period (e.g.,

approximately 24 hours) which triggers the actuator to rotate the rack to a horizontal (flat) position. Alternatively, the racks may be set to rotate at pre-determined times. It should be understood that the eggs may be left in the approximately 45 degree angle for longer than 24 hours and then in the vertical position (i.e., flat rack) for longer than 48 hours. Alternatively, the rack or tray may hold the eggs at a desired first angle when the rack or tray is flat, and then be rotated to an angel such that the eggs are then in a vertical position in the rack or tray. Other, angles for racks and trays may be used. FIG. 6B illustrates an example of eggs positioned in a vertical position 650, in accordance with some embodiments. At this point, the germinal disc should be located at the top (large end) of the egg.

An experiment was conducted to investigate the effectiveness of this protocol 500 on the germinal disc location in an egg. A total of 100 freshly laid fertile eggs were received from a local egg farm over the course of a month. Upon arrival the eggs were stored for three days. Fifty eggs were stored according to the protocol, while the remaining 50 eggs were stored only in the vertical direction with the big end up at the same room temperature for three days. After the three-day storage, the eggshell of each egg was carefully peeled from the center of the egg top (large end) to the side of the boundary defined by the camera's FOV to assess the location of the corresponding germinal disc. For the eggs that were stored as per the protocol, 43 out of 44 eggs (98%) showed the germinal disc right on top of the eggs upon opening and examining the eggs. For the eggs that were stored for three days without positioning at 45 degrees, 38 out of 43 eggs (88%) had their germinal disc on top of the eggs. Thus, the experiment showed that the likelihood of locating germinal disc on top of the egg and in the camera's FOV can be increased by applying the egg handling protocol 500. Therefore, the protocol 500 may improve the performance of egg fertility and gender detection methods that are developed based on detection of light scattering characteristics of the germinal disc.

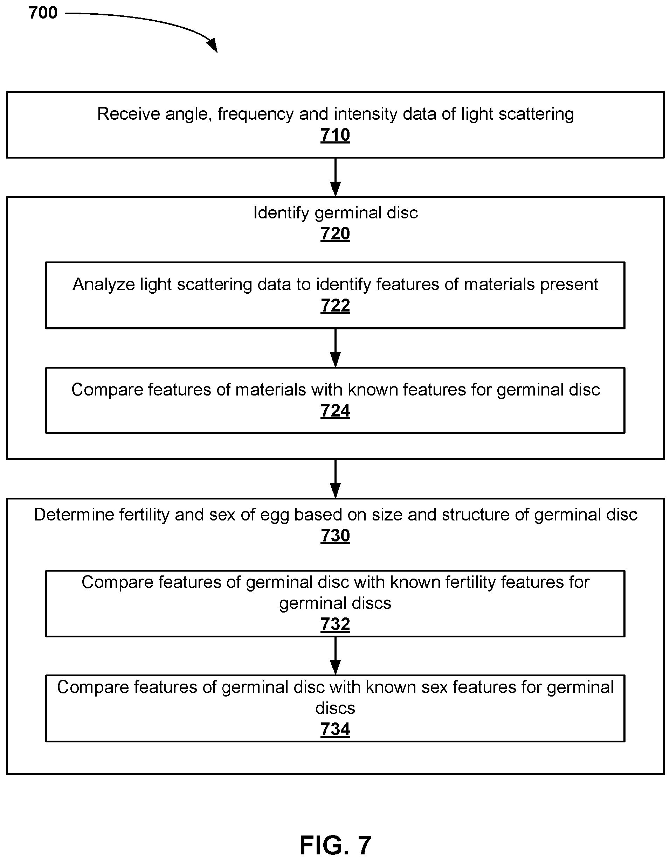

FIG. 7 illustrates, in a flowchart, an example of a method of analyzing light scattering 700, in accordance with some embodiments. The method comprises receiving 710 angle, frequency and intensity data of light scattering obtained from a photon detector 120 from an egg illuminated using a light source 110. For example, eggs may be placed in an egg holder and on a conveyor. The light source 110 may be positioned to align with the photon detector 120 (e.g., HSI camera). The light source 110 will illuminate the egg at the small end, while the big/large end of the egg will be in the FOV of the photon detector. The light scattering data may then be analyzed 720 to identify the germinal disc. The light scattering data is analyzed 722 to identify features of materials present. Next, features (e.g., size, shape, orientation, etc.) of each material in the light scattering data is compared 724 with known features of germinal discs to identify the germinal disc in the light scattering data from any other material or particle present. Next, the fertility and/or sex of the egg is determined **730** based on the size and structure of the identified germinal disc. Features of the identified germinal disc are compared **732** with known fertility features for germinal discs. For example, in a non-fertile egg, the scattering image data of the germinal disc egg may show features that are related to blastodisc which looks like a white pimple; while in a fertile egg, the scattering image data of the germinal disc may show features that are related to blastoderm which looks like a white hollow disc with a white pimple at the centre. Similarly, features of the identified germinal disc are compared **734** with known sex features for germinal discs. Thus, the identified germinal disc data is compared to known germinal disc features to distinguish between fertile and non-fertile eggs, and between male and female eggs. Other steps may be added to the method, including following germinal disc placement protocol **500,** and separating distinguished eggs based on egg type using a conveyor subsystem.

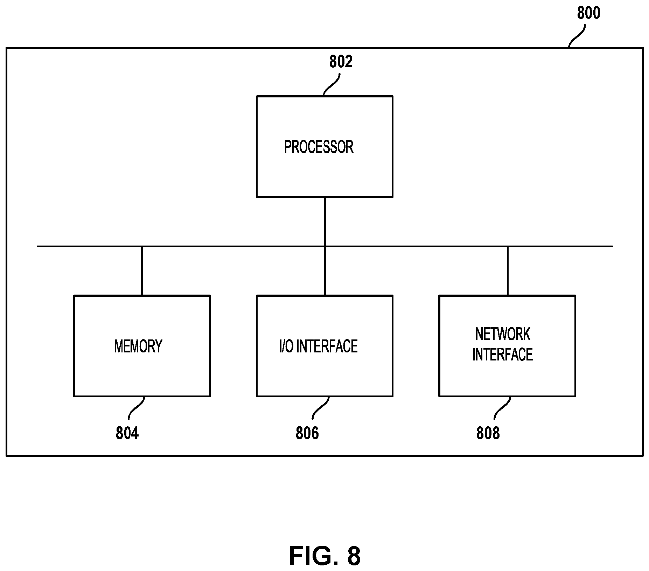

FIG. 8 is a schematic diagram of a computing device **800** such as a server or other computer in a device. As depicted, the computing device includes at least one processor **802,** memory **804,** at least one I/O interface **806,** and at least one network interface **808.**

Processor **802** may be an Intel or AMD x86 or x64, PowerPC, ARM processor, or the like. Memory **804** may include a suitable combination of computer memory that is located either internally or externally such as, for example, random-access memory (RAM), read-only memory (ROM), compact disc read-only memory (CDROM).

Each I/O interface **806** enables computing device **800** to interconnect with one or more input devices, such as a keyboard, mouse, camera, touch screen and a microphone, or with one or more output devices such as a display screen and a speaker.

Each network interface **808** enables computing device **800** to communicate with other components, to exchange data with other components, to access and connect to network resources, to serve applications, and perform other computing applications by connecting to a network (or multiple networks) capable of carrying data including the Internet, Ethernet, plain old telephone service (POTS) line, public switch telephone network (PSTN), integrated services digital network (ISDN), digital subscriber line (DSL), coaxial cable, fiber optics, satellite, mobile, wireless (e.g. Wi-Fi, WiMAX), SS7 signaling network, fixed line, local area network, wide area network, and others.

The embodiments of the devices, systems and methods described herein may be implemented in a combination of both hardware and software. These embodiments may be implemented on programmable computers, each computer including at least one processor, a data storage system (including volatile memory or non-volatile memory or other data storage elements or a combination thereof), and at least one communication interface.

Program code is applied to input data to perform the functions described herein and to generate output information. The output information is applied to one or more output devices. In some embodiments, the communication interface may be a network communication interface. In embodiments in which elements may be combined, the communication interface may be a software communication interface, such as those for inter-process communication. In still other embodiments, there may be a combination of communication interfaces implemented as hardware, software, and combination thereof.

Throughout the foregoing discussion, numerous references will be made regarding servers, services, interfaces, portals, platforms, or other systems formed from computing devices. It should be appreciated that the use of such terms is deemed to represent one or more computing devices having at least one processor configured to execute software instructions stored on a computer readable tangible, non-transitory medium. For example, a server can include one or more computers operating as a web server, database server, or other type of computer server in a manner to fulfill described roles, responsibilities, or functions.

The technical solution of embodiments may be in the form of a software product. The software product may be stored in a non-volatile or non-transitory storage medium, which can be a compact disk read-only memory (CD-ROM), a USB flash disk, or a removable hard disk. The software product includes a number of instructions that enable a computer device (personal computer, server, or network device) to execute the methods provided by the embodiments.

The embodiments described herein are implemented by physical computer hardware, including computing devices, servers, receivers, transmitters, processors, memory, displays, and networks. The embodiments described herein provide useful physical machines and particularly configured computer hardware arrangements.

Although the embodiments have been described in detail, it should be understood that various changes, substitutions and alterations can be made herein.

As can be understood, the examples described above and illustrated are intended to be exemplary only.

## Claims

1. An egg analysis system (100, 150, 200, 250) comprising:

   at least one light source (110, 115, 210, 260) for illuminating an egg (225) by emitting light that covers the field of view, FOV, of a hyperspectral imaging camera (115, 120, 230);

the hyperspectral imaging camera (115, 120, 230) for detecting a fluctuation of scattering light emitted from the egg (225);

at least one processor (130, 802); and

a memory (140, 804) storing instructions which, when executed by the at least one processor (130, 802), configure the at least one processor (130, 802) to:

actuate the at least one light source (110, 115, 210, 260) to illuminate the egg (225);

receive, from the hyperspectral imaging camera (115, 120, 230), scattering light data comprising angle, frequency and intensity data;

digitize the scattering light data;

analyze the digitized scattering light data to identify features of material present;

compare the identified features of material present with known features for germinal discs, to identify the germinal disc in the light scattering data; and

determine at least one of a fertility or a sex of the egg (225) based on the size and structure of the germinal disc.

2. The system (100, 150, 200, 250) as claimed in claim 1, wherein the FOV is approximately 16 centimeters x 2.5 centimeters.

3. The system (100, 150, 200, 250) as claimed in claim 1, wherein the at least one light source (110, 115, 210, 260) emits a wavelength range that is at least 120 mW·sr$^{-1}$·cm$^{-2}$·nm$^{-1}$ for spectral intensity.

4. The system (100, 150, 200, 250) as claimed in claim 1, wherein the at least one light source (110, 115, 210, 260) provides stability for a period of approximately 8 hours or above.

5. The system (100, 150, 200, 250) as claimed in claim 4, wherein the at least one light source (110, 115, 210, 260) comprises a lamp having a lifetime of greater than 1,000 hours.

6. The system (100, 150, 200, 250) as claimed in claim 1, wherein the at least one light source (110, 115, 210, 260) comprises two or more halogen lamps wherein each halogen lamp comprises:

a 250 W QTH lamp;

a light source housing;

a lamp power supply;

a fiber bundle focusing assembly;

a liquid light guide (215); and

a light guide collimating probe.

7. The system (100, 150, 200, 250) of claim 1, wherein the at least one light source (110, 115, 210, 260) comprises one or more light emitting diodes, LEDs.

8. The system (100, 150, 200, 250) as claimed in claim 7, wherein each of the one or more LEDs emits light at a wavelength comprising one of approximately:

860 nanometers;

1050 nanometers; or

1250 nanometers.

9. The system (100, 150, 200, 250) as claimed in claim 1, wherein to identify the germinal disc, the at least one processor (130, 802) is further configured to:

receive the egg (225) in a tray;

actuate the tray to position the egg (225) at a first angle for a first period of time, with a large end of the egg (225) higher than a small end of the egg (225); and

actuate the tray to position the egg (225) in a vertical position with the large end up for a second period of time.

10. The system (100, 150, 200, 250) as claimed in claim 9, wherein at least one of:

the first angle is approximately 45 degrees;

the first period is approximately 24 hours; or

the second period is approximately 48 hours.

**Patentansprüche**

1. Eianalysesystem (100, 150, 200, 250), umfassend:

mindestens eine Lichtquelle (110, 115, 210, 260) zum Beleuchten eines Eies (225) durch Licht, das das Sichtfeld, FOV, einer hyperspektralen Bildgebungskamera (115, 120, 230) abdeckt;

die hyperspektrale Bildgebungskamera (115, 120, 230) zum Erfassen einer Fluktuation von Streulicht konfiguriert ist, das von dem Ei (225) emittiert wird;

mindestens einen Prozessor (130, 802); und

einen Speicher (140, 804), der Anweisungen speichert, die bei Ausführung durch den mindestens einen Prozessor (130, 802), den mindestens einen Prozessor (130, 802) konfigurieren, zum:

Ansteuern der mindestens einen Lichtquelle (110, 115, 210, 260), um das Ei (225) zu beleuchten; Empfangen, von der hyperspektralen Bildgebungskamera (115, 120, 230), von Streulichtdaten, die Winkel-, Frequenz- und Intensitätsdaten umfassen;

Digitalisieren der Streulichtdaten;
Analysieren der digitalisierten Streulichtdaten, um Merkmale des vorhandenen Materials zu identifizieren;
Vergleichen der identifizierten Merkmale des vorhandenen Materials mit bekannten Merkmalen für Keimscheiben, um die Keimscheibe in den Lichtstreudaten zu identifizieren; und
Bestimmen mindestens eines von einer Fertilität oder eines Geschlechts des Eies (225) basierend auf der Größe und Struktur der Keimscheibe.

2. System (100, 150, 200, 250) nach Anspruch 1, wobei das FOV ungefähr 16 Zentimeter x 2,5 Zentimeter beträgt.

3. System (100, 150, 200, 250) nach Anspruch 1, wobei die mindestens eine Lichtquelle (110, 115, 210, 260) einen Wellenlängenbereich emittiert, der mindestens 120 mW·sr$^{-1}$·cm$^{-2}$·nm$^{-1}$ für spektrale Intensität beträgt.

4. System (100, 150, 200, 250) nach Anspruch 1, wobei die mindestens eine Lichtquelle (110, 115, 210, 260) Stabilität für einen Zeitraum von etwa 8 Stunden oder mehr bereitstellt.

5. System (100, 150, 200, 250) nach Anspruch 4, wobei die mindestens eine Lichtquelle (110, 115, 210, 260) eine Lampe mit einer Lebensdauer von mehr als 1.000 Stunden umfasst.

6. System (100, 150, 200, 250) nach Anspruch 1, wobei die mindestens eine Lichtquelle (110, 115, 210, 260) zwei oder mehr Halogenlampen umfasst, wobei jede Halogenlampe umfasst:

eine 250 W QTH-Lampe;
ein Lichtquellengehäuse;
eine Stromversorgung für die Lampe;
eine Faserbündel-Fokussieranordnung;
einen flüssigen Lichtleiter (215); und
eine Lichtleiter-Kollimationssonde.

7. System (100, 150, 200, 250) nach Anspruch 1, wobei die mindestens eine Lichtquelle (110, 115, 210, 260) eine oder mehrere lichtemittierende Dioden (LEDs) umfasst.

8. System (100, 150, 200, 250) nach Anspruch 7, wobei jede der einen oder mehreren LEDs Licht mit einer Wellenlänge emittiert, die eine von etwa:

860 Nanometern;
1050 Nanometer; oder
1250 Nanometer ist.

9. System (100, 150, 200, 250) nach Anspruch 1, wobei zur Identifizierung der Keimscheibe der mindestens eine Prozessor (130, 802) ferner konfiguriert ist, zum:

Aufnehmen des Eis (225) in einer Schale;
Ansteuern der Schale, um das Ei (225) für eine erste Zeitspanne in einem ersten Winkel zu positionieren, wobei ein großes Ende des Eies (225) höher liegt als ein kleines Ende des Eies (225); und
Ansteuern der Schale, um das Ei (225) für eine zweite Zeitspanne in einer vertikalen Position mit dem großen Ende nach oben zu positionieren.

10. System (100, 150, 200, 250) nach Anspruch 9, wobei mindestens eines der folgenden gilt:

der erste Winkel beträgt etwa 45 Grad;
die erste Zeitspanne beträgt etwa 24 Stunden; oder
die zweite Zeitspanne beträgt etwa 48 Stunden.

**Revendications**

1. Système d'analyse d'œuf (100, 150, 200, 250), comprenant :

au moins une source lumineuse (110, 115, 210, 260) destinée à éclairer un œuf (225) en émettant de la lumière qui couvre le champ de vision, FOV, d'une caméra d'imagerie hyperspectrale (115, 120, 230);
la caméra d'imagerie hyperspectrale (115, 120, 230) étant destinée à détecter une fluctuation de la lumière de diffusion émise par l'œuf (225);
au moins un processeur (130, 802); et
une mémoire (140, 804) stockant des instructions qui, lorsqu'elles sont exécutées par l'au moins un processeur (130, 802), configurent l'au moins un processeur (130, 802) pour :

actionner l'au moins une source lumineuse (110, 115, 210, 260) pour éclairer l'œuf (225);
recevoir, en provenance de la caméra d'imagerie hyperspectrale (115, 120, 230), des données de lumière de diffusion comprenant des données d'angle, de fréquence et d'intensité ;
numériser les données de lumière de diffusion ;
analyser les données de lumière de diffusion numérisées pour identifier les caractéristiques du matériau présent ;
comparer les caractéristiques identifiées du matériau présent à des caractéristiques connues pour des disques germinaux, afin d'identifier le disque germinal dans les données de lumière de diffusion ; et déterminer au moins l'un(e) parmi la fertilité ou le sexe de l'œuf (225) sur la base de la taille et de la structure du disque germinal.

2. Système (100, 150, 200, 250) tel que revendiqué dans la revendication 1, dans lequel le FOV est d'environ 16 centimètres x 2,5 centimètres.

3. Système (100, 150, 200, 250) tel que revendiqué dans la revendication 1, dans lequel l'au moins une source lumineuse (110, 115, 210, 260) émet une gamme de longueurs d'onde dont l'intensité spectrale est d'au moins 120 mW·sr$^{-1}$·cm$^{-2}$·nm$^{-1}$.

4. Système (100, 150, 200, 250) tel que revendiqué dans la revendication 1, dans lequel l'au moins une source lumineuse (110, 115, 210, 260) assure la stabilité sur une période d'environ 8 heures ou plus.

5. Système (100, 150, 200, 250) tel que revendiqué dans la revendication 4, dans lequel l'au moins une source lumineuse (110, 115, 210, 260) comprend une lampe ayant une durée de vie supérieure à 1 000 heures.

6. Système (100, 150, 200, 250) tel que revendiqué dans la revendication 1, dans lequel l'au moins une source lumineuse (110, 115, 210, 260) comprend deux lampes halogènes ou plus, dans laquelle chaque lampe halogène comprend :

    une lampe QTH de 250 W;
    un boîtier de source lumineuse ;
    une alimentation électrique de lampe ;
    un ensemble de focalisation de faisceau de fibres ;
    un guide de lumière liquide (215); et
    une sonde de collimation de guide de lumière.

7. Système (100, 150, 200, 250) de la revendication 1, dans lequel l'au moins une source lumineuse (110, 115, 210, 260) comprend une ou plusieurs diodes électroluminescentes, LED.

8. Système (100, 150, 200, 250) tel que revendiqué dans la revendication 7, dans lequel chacune de la ou des LED émet de la lumière à une longueur d'onde comprenant l'une des valeurs approximatives suivantes :

    860 nanomètres ;
    1 050 nanomètres ; ou
    1 250 nanomètres.

9. Système (100, 150, 200, 250) tel que revendiqué dans la revendication 1, dans lequel, pour identifier le disque germinatif, l'au moins un processeur (130, 802) est en outre configuré pour :

    recevoir l'œuf (225) sur un plateau ;
    actionner le plateau afin de positionner l'œuf (225) selon un premier angle pendant une première période de temps, la grande extrémité de l'œuf (225) étant plus haute que la petite extrémité de l'œuf (225) ; et
    actionner le plateau afin de positionner l'œuf (225) en position verticale, la grande extrémité vers le haut, pendant une seconde période de temps.

10. Système (100, 150, 200, 250) tel que revendiqué dans la revendication 9, dans lequel au moins l'un parmi :

    le premier angle est d'environ 45 degrés ;
    la première période dure environ 24 heures ; ou
    la seconde période dure environ 48 heures.

Egg Analysis System
**100**

Light Source
**110**

Photon
Detector
**120**

Processor
**130**

Memory
**140**

## FIG. 1A

Egg Analysis System
**150**

Imaging
Subsystem
**115**

Detection
Subsystem
**125**

Actuating
Subsystem
**105**

## FIG. 1B

200

Detector
230

Egg
225

Focused beam

Lens
Lens
Lens
220

Optical fiber/LLG
215

Light source
210

**FIG. 2A**

250

Detector
230

Egg
225

Focused line light

Lens
270

LED line light
260

**FIG. 2B**

FIG. 3A   FIG. 3B   FIG. 3C   FIG. 3D   FIG. 3E

400

```
┌─────────────────────────────────────────────┐
│              Illuminate egg                  │
│                    410                       │
└─────────────────────────────────────────────┘
                      │
                      ▼
┌─────────────────────────────────────────────┐
│    Detect fluctuation of scattering light    │
│                  emitted                     │
│                    420                       │
└─────────────────────────────────────────────┘
                      │
                      ▼
┌─────────────────────────────────────────────┐
│  Digitize detect fluctuation of scattering   │
│                   light                      │
│                    430                       │
└─────────────────────────────────────────────┘
                      │
                      ▼
┌─────────────────────────────────────────────┐
│ Analyze digitized fluctuation of scattering  │
│                   light                      │
│                    440                       │
└─────────────────────────────────────────────┘
```

## FIG. 4

500

```
┌─────────────────────────────────────────────┐
│               Receive eggs                   │
│                    510                       │
└─────────────────────────────────────────────┘
                      │
                      ▼
┌─────────────────────────────────────────────┐
│           Store eggs for three days          │
│                    520                       │
│  ┌───────────────────────────────────────┐  │
│  │ Position eggs at 45 degree angle for   │  │
│  │               24 hours                 │  │
│  │                 522                    │  │
│  └───────────────────────────────────────┘  │
│                    │                         │
│                    ▼                         │
│  ┌───────────────────────────────────────┐  │
│  │    Position eggs vertically for 48     │  │
│  │               hours                    │  │
│  │                 524                    │  │
│  └───────────────────────────────────────┘  │
└─────────────────────────────────────────────┘
```

## FIG. 5

FIG. 6B

650

FIG. 6A

600

700

Receive angle, frequency and intensity data of light scattering
**710**

Identify germinal disc
**720**

Analyze light scattering data to identify features of materials present
**722**

Compare features of materials with known features for germinal disc
**724**

Determine fertility and sex of egg based on size and structure of germinal disc
**730**

Compare features of germinal disc with known fertility features for germinal discs
**732**

Compare features of germinal disc with known sex features for germinal discs
**734**

# FIG. 7

800

802

PROCESSOR

MEMORY

I/O INTERFACE

NETWORK
INTERFACE

804

806

808

# FIG. 8

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 214041128
- KR 20180055970
- CA 2804903
- EP 4260048 A